# EUROPÄISCHE PATENTSCHRIFT

(11) **EP 1 902 035 B1**
(45) Veröffentlichungstag und Bekanntmachung des Hinweises auf die Patenterteilung: **11.04.2012**
(21) Anmeldenummer: 06754375.1
(22) Anmeldetag: 14.06.2006
(51) Int. Cl.: C07D 233/54

(54) **VERFAHREN ZUR HERSTELLUNG VON ONIUM-ALKYLSULFITEN**
METHOD FOR THE PRODUCTION OF ONIUM ALKYL SULFITES
PROCEDE DE PREPARATION D'ALKYLSULFITES D'ONIUM

(30) Priorität: 14.07.2005 DE 102005032837
(43) Veröffentlichungstag der Anmeldung: 26.03.2008
(73) Patentinhaber: Merck Patent GmbH, 64293 Darmstadt (DE)
(72) Erfinder: IGNATYEV, Nikolai (Mykola), 47058 Duisburg (DE); WELZ-BIERMANN, Urs, 64646 Heppenheim (DE); KUCHERYNA, Andriy, Kyiv 4215 (UA); WILLNER, Helge, 45481 Mühlheim/Ruhr (DE)
(86) Internationale Anmeldenummer: PCT/EP2006/005742
(87) Internationale Veröffentlichungsnummer: WO 2007/006388

(56) Entgegenhaltungen:
- WO-A-02/30878
- WO-A-02/34722
- WO-A-96/18459
- WO-A-2006/021302
- WO-A-2006/021303
- WO-A-2006/021304
- US-A- 3 637 622
- DATABASE WPI Week 1988 Derwent Publications Ltd., London, GB; AN 1988-356115 XP002401878 & JP 63 262830 A (MITSUBISHI PETROCHEMICAL CO LTD) 31. Oktober 1988 (1988-10-31)
- DATABASE WPI Week 2002 2002, Derwent Publications Ltd., London, GB; AN 2002-419496 XP002402094 & JP 2001 322970 A (TONEN KAGAKU KK) 20. November 2001 (2001-11-20)
- MALLION, K. B., MANN, FREDERICK G.: "The Conditions Determining the Quaternisation of Tertiary Phosphines by Methyl 2,4-Dinitrosulphonate" J. CHEM. SOC., SUPPL., Bd. 1964, Nr. 1, 1964, Seiten 5716-5725, XP009073419

## Beschreibung

Die Erfindung betrifft ein Verfahren zur Herstellung von Onium-Alkylsulfiten durch Umsetzung eines Onium-Halogenids oder -Carboxylats mit einem symmetrisch substituierten Dialkylsulfit oder mit einem unsymmetrisch substituierten Dialkylsulfit bei Temperaturen von 0 bis 70°C sowie mit diesem Verfahren hergestellte Alkylsulfite.

Eine große Anzahl von Onium-Salzen, beispielsweise Alkylsulfate, sind ionische Flüssigkeiten. Ionische Flüssigkeiten stellen durch Ihre Eigenschaften in der modernen Forschung eine wirksame Alternative zu traditionellen flüchtigen organischen Solventien für die organische Synthese dar. Die Verwendung von ionischen Flüssigkeiten als neues Reaktionsmedium könnte weiterhin eine praktische Lösung sowohl für die Lösungsmittel Emission als auch für Probleme in der Wiederaufbereitung von Katalysatoren sein (R. Sheldon, Chem. Commun., 2001, S. 2399-2407).

Ionische Flüssigkeiten oder flüssige Salze sind ionische Spezies, die aus einem organischen Kation und einem in der Regel anorganischen Anion bestehen. Sie enthalten keine neutralen Moleküle und weisen meistens Schmelzpunkte kleiner 373 K auf. Der Schmelzpunkt kann jedoch auch höher liegen, ohne die Anwendbarkeit der Salze auf allen Anwendungsgebieten zu beschränken. Beispiele für organische Kationen sind unter anderem Tetraalkylammonium, Tetraalkylphosphonium, N-Alkylpyridinium, 1,3-Dialkylimidazolium oder Trialkylsulfonium. Unter einer Vielzahl von geeigneten Anionen sind beispielsweise BF₄⁻, PF₆⁻, SbF₆⁻, NO₃⁻, CF₃SO₃⁻, (CF₃SO₂)₂N⁻, AlkylSO₃⁻, ArylSO₃⁻, CF₃CO₂⁻, CH₃CO₂⁻ oder Al₂Cl₇⁻ zu nennen.

Vereinzelte Onium-Salze mit Methylsulfit-Anion sind in der Literatur bschrieben, z.B. Trimethyl-phenyl-ammonium-methylsulfit (W. Voss und E. Blanke, Justus Liebigs Ann. Chem., 485 (1931), S. 258-279) oder Tetrabutylammonium-methylsulfit (M. Julia et al., Tetrahedron, 42 (1986), S. 3841-3850). Darüber hinaus gibt es keine generelle Methode zur Herstellung von Onium-Alkylsulfiten.

JP 63262830 beschreibt Elektrolyte für Kondensatoren enthaltend Ammoniumsalze oder quartemäre Phosphoniumsalze der schwefligen Säure oder Monoester der schwefligen Säure. Als Beispiele für Kationen werden unter anderem N-Ethylpyridinium, N,N'-Dimehtylimidazolium genannt.

US 3,637,622 beschreibt ein Verfahren zur Polymerisation von Vinylchlorid, wobei als Katalysator ein Ammoniumsalz eines Monoesters der schwefligen Säure eingesetzt werden kann.

K.B. Mallon et al, J.Chem. Soc. Suppl, Vol. 1964, 1, 1964, Seiten 5716-5725 beschreibt die Quarternisierung von tertiären Phosphinen zu Methylphosphonium-Salzen, wobei unter Formel (V) ein Phosphoniummethylsulfit beschrieben wird.

Die nachveröffentlichten Dokumente WO 2006/021302 und WO 2006/021304 beschreiben Verfahren zur Herstellung quartärer Ammoniumverbindungen durch Umsetzung eines Amins oder eines Imins mit Dimethylsulfit.

Das nachveröffentlichte Dokument WO 2006/021303 beschreibt Imidazolium-Methylsulfite als Ausgangsverbindungen zur Herstellung ionischer Flüssigkeiten.

Aufgabe der vorliegenden Erfindung war dementsprechend ein Verfahren zur Herstellung von Onium-Alkylsulfiten zur Verfügung zu stellen, welches zu Alkylsulfiten mit frei wählbarem Substitutionsmuster bei Kation und Anion führt, sowie die Bereitstellung von Onium-Alkylsulfiten.

Die Aufgabe wird durch das erfindungsgemäße Verfahren gelöst. Gegenstand der Erfindung ist daher ein Verfahren zur Herstellung von Onium-Alkylsulfiten durch Umsetzung eines Onium-Halogenids oder Onium-Carboxylats mit einem symmetrisch substituierten Dialkylsulfit oder mit einem unsymmetrisch substituierten Dialkylsulfit bei Temperaturen von 0 bis 70°C. Das erfindungsgemäße Verfahren hat den Vorteil, dass keine Alkylgruppe des eingesetzten Sulfits auf das Kation übertragen wird und damit das Substitutionsmuster im Kation von der Wahl des Alkylsulfitanions unabhängig ist. Darüber hinaus sind die eingesetzten Dialkylsulfite weniger toxisch als Alkylsulfatester. Als Nebenprodukte des erfindungsgemäßen Verfahrens werden Alkylhalogenide oder Ester erhalten, die ihrerseits als wertvolle Reagenzien eingesetzt werden können. Die bei Verwendung von Onium-Halogeniden als Nebenprodukt entstehenden Alkylhalogenide sind darüber hinaus in der Regel Gase oder leicht flüchtige Verbindungen, die ohne großen prozesstechnischen Aufwand aus der Reaktionsmischung entfernt werden können

Selbstverständlich ist der Einsatz sowohl von symmetrisch substituierten Dialkylsulfiten mit 1 bis 10 C-Atomen oder der Einsatz von unsymmetrisch substituierten Dialkylsulfiten mit 1 bis 10 C-Atomen oder noch höher alkylierten Edukten im erfindungsgemäßen Verfahren möglich.

Für das erfindungsgemäße Verfahren geeignete Onium-Halogenide sind Ammoniumhalogenide, Phosphoniumhalogenide, Thiouroniumhalogenide, Guanidiniumhalogenide oder Halogenide mit heterocyclischem Kation, wobei die Halogenide aus der Gruppe Fluoride, Chloride oder Bromide ausgewählt werden können. Bevorzugt werden im erfindungsgemäßen Verfahren Ammonium, Phosphonium-, Guanidiniumhalogenide oder Halogenide mit heterocyclischem Kation eingesetzt.

Für das erfindungsgemäße Verfahren geeignete Onium-Carboxylate sind Ammoniumcarboxylate, Phosphoniumcarboxylate, Thiouroniumcarboxylate, Guanidiniumcarboxylate oder Carboxylate mit heterocyclischem Kation, wobei die Carboxylate aus der Gruppe der Carbonate, Formiate, Acetate, Propylate oder Butylate ausgewählt werden können. Bevorzugt werden im erfindungsgemäßen Verfahren Ammonium-, Phosphonium-, Guanidiniumcarboxylate oder Carboxylate mit heterocyclischem Kation eingesetzt, insbesondere Ammonium-, Phosphonium-, Guanidiniumacetate oder Acetate mit heterocyclischem Kation.

Bevorzugt werden Onium-Halogenide in den erfindungsgemäßen Verfahren eingesetzt.

Die Onium-Halogenide oder -Carboxylate sind in der Regel kommerziell erhältlich oder können nach Syntheseverfahren hergestellt werden, wie sie aus der Literatur, z.B. in den Standardwerken wie Houben-Weyl, Methoden der organischen Chemie, Georg-Thieme-Verlag, Stuttgart oder Richard C. Larock, Comprehensive Organic Transformations, 2nd Edition, Wiley-VCH, New York, 1999 bekannt sind. Dabei kann man auch von an sich bekannten, hier nicht näher erwähnten Varianten Gebrauch machen.

Ammonium- oder Phosphoniumhalogenide können beispielsweise durch die Formel (1)
beschrieben werden,

[XR₄]⁺ Hal⁻ (1),

wobei
X N, P
Hal F, Cl oder Br und
R jeweils unabhängig voneinander
H, wobei nicht alle Substituenten R gleichzeitig H sein dürfen, geradkettiges oder verzweigtes Alkyl mit 1-20 C-Atomen,
geradkettiges oder verzweigtes Alkenyl mit 2-20 C-Atomen und einer oder mehreren Doppelbindungen bedeutet,
geradkettiges oder verzweigtes Alkinyl mit 2-20 C-Atomen und einer oder mehreren Dreifachbindungen,
gesättigtes, teilweise oder vollständig ungesättigtes Cycloalkyl mit 3-7 C-Atomen, das mit Alkylgruppen mit 1-6 C-Atomen substituiert sein kann bedeutet,
wobei ein oder mehrere R teilweise mit Cl, Br und/oder CN oder teilweise oder vollständig mit -F oder F und Cl, oder F und Br, oder F, Cl und Br substituiert sein können, wobei jedoch nicht alle vier oder drei R vollständig mit Halogenen substituiert sein dürfen,
und wobei ein oder zwei nicht benachbarte und nicht α- oder w-ständige Kohlenstoffatome des R, durch Atome und/oder Atomgruppierungen ausgewählt aus der Gruppe -O-, -C(O)-, -S-, -S(O)- oder -SO₂- ersetzt sein können.

Ausgeschlossen sind demnach Verbindungen der Formel (1), in denen alle vier oder drei Substituenten R vollständig mit Halogenen substituiert sind, beispielsweise Tris(trifluormethyl)methylammoniumchlorid, Tetra(trifluormethyl)ammoniumchlorid oder Tetra(nonafluorbutyl)phosphoniumchlorid.

Guanidiniumhalogenide können beispielsweise durch die Formel (2) beschrieben werden,

[C(NR¹R²)(NR³R⁴)(NR⁵R⁶)]⁺ Hal⁻ (2),

wobei
Hal F, Cl oder Br und
R¹ bis R⁶ jeweils unabhängig voneinander
Wasserstoff oder CN,
geradkettiges oder verzweigtes Alkyl mit 1 bis 20 C-Atomen, geradkettiges oder verzweigtes Alkenyl mit 2-20 C-Atomen und einer oder mehreren Doppelbindungen,
geradkettiges oder verzweigtes Alkinyl mit 2-20 C-Atomen und einer oder mehreren Dreifachbindungen,
gesättigtes, teilweise oder vollständig ungesättigtes Cycloalkyl mit 3-7 C-Atomen, das mit Alkylgruppen mit 1-6 C-Atomen substituiert sein kann, bedeutet,
wobei ein oder mehrere der Substituenten R¹ bis R⁶ teilweise mit -NO₂, CN, Cl und/oder Br, teilweise oder vollständig mit F, oder F und Cl, oder F und Br oder F, Cl und Br substituiert sein können, wobei jedoch nicht alle Substituenten an einem N-Atom vollständig mit Halogenen substituiert sein dürfen,
wobei die Substituenten R¹ bis R⁶ paarweise durch Einfach- oder Doppelbindung miteinander verbunden sein können und
wobei ein oder zwei nicht benachbarte, nicht direkt am Heteroatom gebundene und nicht ω-ständige Kohlenstoffatome der Substituenten R¹ bis R⁶ durch Atome und/oder Atomgruppierungen ausgewählt aus der Gruppe -O-, -C(O)-, -S-, -S(O)- oder -SO₂- ersetzt sein können.

Thiouroniumhalogenide können beispielsweise durch die Formel (3)

[(R¹R²N)-C(=SR⁷)(NR³R⁴)]⁺ Hal⁻ (3)

beschrieben werden,
wobei
Hal Cl oder Br und
R¹ bis R⁴ und R⁷ jeweils unabhängig voneinander
Wasserstoff oder CN, wobei Wasserstoff für R⁷ ausgeschlossen wird, geradkettiges oder verzweigtes Alkyl mit 1 bis 20 C-Atomen, geradkettiges oder verzweigtes Alkenyl mit 2-20 C-Atomen und einer oder mehreren Doppelbindungen,
geradkettiges oder verzweigtes Alkinyl mit 2-20 C-Atomen und einer oder mehreren Dreifachbindungen,
gesättigtes, teilweise oder vollständig ungesättigtes Cycloalkyl mit 3-7 C-Atomen, das mit Alkylgruppen mit 1-6 C-Atomen substituiert sein kann, bedeutet,
wobei ein oder mehrere der Substituenten R¹ bis R⁴ und R⁷ teilweise oder vollständig mit F, Cl und/oder Br, insbesondere -F und/oder -Cl, oder teilweise mit CN substituiert sein können, wobei jedoch nicht alle Substituenten an einem N-Atom vollständig mit Halogenen substituiert sein dürfen,
wobei die Substituenten R¹ bis und R⁷ paarweise durch Einfach- oder Doppelbindung miteinander verbunden sein können und wobei ein oder zwei nicht benachbarte, nicht direkt am Heteroatom gebundene und nicht ω-ständige Kohlenstoffatome der Substituenten R¹ bis und R⁷ durch Atome und/oder Atomgruppierungen ausgewählt aus der Gruppe -O-, -C(O)-, -S-, -S(O)- oder -SO₂- ersetzt sein können.

Halogenide mit heterocyclischem Kation können beispielsweise durch die Formel (4) beschrieben werden,

[HetN]⁺ Hal⁻ (4),

wobei
Hal Cl oder Br und
HetN⁺ ein heterocyclisches Kation, ausgewählt aus der Gruppe 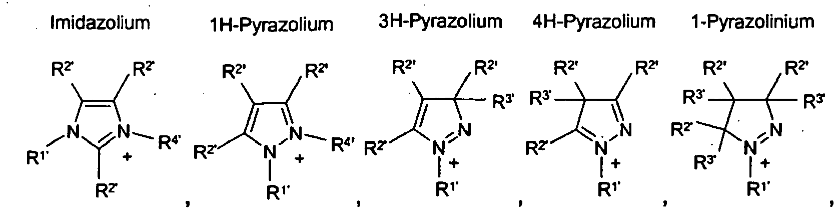 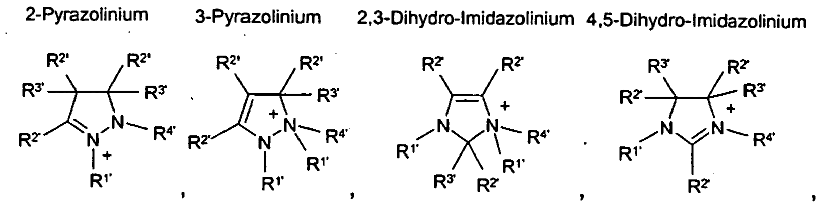 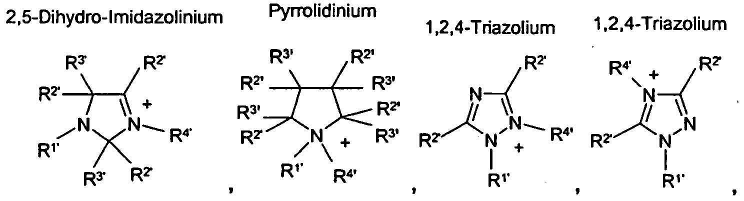 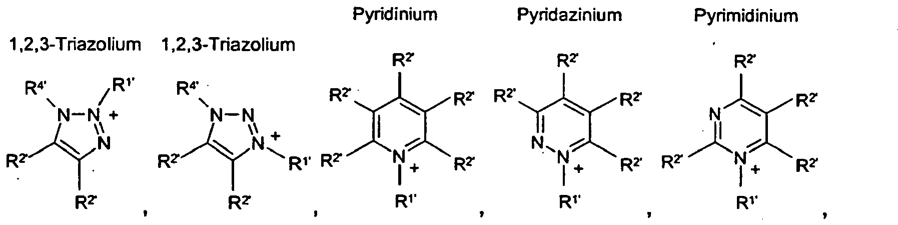 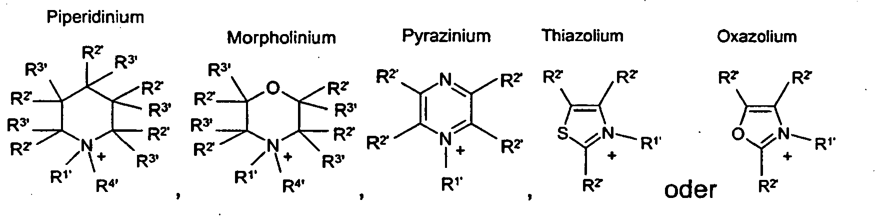 bedeutet, wobei die Substituenten
R^{1,} bis R^{4,} jeweils unabhängig voneinander
Wasserstoff oder CN,
geradkettiges oder verzweigtes Alkyl mit 1-20 C-Atomen,
geradkettiges oder verzweigtes Alkenyl mit 2-20 C-Atomen und einer oder mehreren Doppelbindungen,
geradkettiges oder verzweigtes Alkinyl mit 2-20 C-Atomen und einer oder mehreren Dreifachbindungen,
Dialkylamino mit Alkylgruppen mit 1-4 C-Atomen, welches jedoch nicht am Heteroatom des Heterocyclus gebunden vorliegt,
gesättigtes, teilweise oder vollständig ungesättigtes Cycloalkyl mit 3-7 C-Atomen, das mit Alkylgruppen mit 1-6 C-Atomen substituiert sein kann oder Aryl-C₁-C₆-alkyl bedeutet,
wobei die Substituenten R^{1,} und R^{4,} teilweise oder vollständig mit F, Cl und/oder Br, insbesondere -F und/oder -Cl substituiert sein können, wobei jedoch nicht gleichzeitig R^{1'} und R^{4'} CN sind oder vollständig mit F oder anderen Halogenen substituiert sein dürfen,
wobei die Substituenten R^{2'} und R^{3'} teilweise oder vollständig mit F, Cl und/oder Br, insbesondere -F und/oder -Cl, oder teilweise mit NO₂ oder CN substituiert sein dürfen
und wobei ein oder zwei nicht benachbarte, nicht direkt am Heteroatom gebundene und nicht ω-ständige Kohlenstoffatome der Substituenten R^{1,} bis R^{4,}, durch Atome und/oder Atomgruppierungen ausgewählt aus der Gruppe -O-, -C(O)-, -S-, -S(O)- oder -SO₂- ersetzt sein können.

Die C₁-C₂₀-Alkylgruppe ist beispielsweise Methyl, Ethyl, Isopropyl, Propyl, Butyl, sek.-Butyl oder tert.-Butyl, ferner auch Pentyl, 1-, 2- oder 3-Methylbutyl, 1,1-, 1,2- oder 2,2-Dimethylpropyl, 1-Ethylpropyl, Hexyl, Heptyl, Octyl, Nonyl, Decyl, Undecyl, Dodecyl, Tridecyl oder Tetradecyl. Gegebenenfalls fluorierte Alkylgruppen, beispielsweise Difluormethyl, Trifluormethyl, Tetrafluoroethyl, Pentafluorethyl, Heptafluorpropyl oder Nonafluorbutyl.

Ein geradkettiges oder verzweigtes Alkenyl mit 2 bis 20 C-Atomen, wobei auch mehrere Doppelbindungen vorhanden sein können, ist beispielsweise Vinyl, Allyl, 2- oder 3-Butenyl, Isobutenyl, sek.-Butenyl, ferner 4-Pentenyl, iso-Pentenyl, Hexenyl, Heptenyl, Octenyl, -C₉H₁₇, -C₁₀H₁₉ bis -C₂₀H₃₉; vorzugsweise Vinyl, Allyl, 2- oder 3-Butenyl, Isobutenyl, sek.-Butenyl, ferner bevorzugt ist 4-Pentenyl, iso-Pentenyl oder Hexenyl.

Ein geradkettiges oder verzweigtes Alkinyl mit 2 bis 20 C-Atomen, wobei auch mehrere Dreifachbindungen vorhanden sein können, ist beispielsweise Ethinyl, 1- oder 2-Propinyl, 2- oder 3-Butinyl, ferner 4-Pentinyl, 3-Pentinyl, Hexinyl, Heptinyl, Octinyl, -C₉H₁₅, -C₁₀H₁₇ bis -C₂₀H₃₇, vorzugsweise Ethinyl, 1- oder 2-Propinyl, 2- oder 3-Butinyl, 4-Pentinyl, 3-Pentinyl oder Hexinyl.

Unter vollständig ungesättigtem Cycloalkyl werden im Sinne der vorliegenden Erfindung auch aromatische Substituenten verstanden.

Unsubstituierte gesättigte oder teilweise oder vollständig ungesättigte Cycloalkylgruppen mit 3-7 C-Atomen sind daher Cyclopropyl, Cyclobutyl, Cyclopentyl, Cyclohexyl, Cycloheptyl, Cyclopentenyl, Cyclopenta-1,3-dienyl, Cyclohexenyl, Cyclohexa-1,3-dienyl, Cyclohexa-1,4-dienyl, Phenyl, Cycloheptenyl, Cyclohepta-1,3-dienyl, Cyclohepta-1,4-dienyl oder Cyclohepta-1,5-dienyl, welche mit C₁- bis C₆-Alkylgruppen substituiert sein können, wobei wiederum die Cycloalkylgruppe oder die mit C₁- bis C₆-Alkylgruppen substituierte Cycloalkylgruppe auch mit F oder F und Cl substituiert sein kann. Die Cycloalkylgruppen können jedoch ebenfalls durch weitere funktionelle Gruppen substituiert sein, beispielsweise durch CN, SO₂R', SO₂OR', SO₂NH₂, C(O)NH₂ oder C(O)OR'. R' hat dabei eine nachfolgend definierte Bedeutung.

Aryl-C₁-C₆-alkyl bedeutet beispielsweise Benzyl, Phenylethyl, Phenylpropyl, Phenylbutyl, Phenylpentyl oder Phenylhexyl, wobei sowohl der Phenylring als auch die Alkylenkette, wie zuvor beschrieben teilweise oder vollständig mit -F, oder F und Cl substituiert sein können, besonders bevorzugt Benzyl oder Phenylpropyl. Der Phenylring oder auch die Alkylenkette können jedoch ebenfalls durch weitere funktionelle Gruppen substituiert sein, beispielsweise durch CN, SO₂R', SO₂OR', SO₂NH₂, C(O)NH₂ oder C(O)OR'.

R' bedeutet nicht, teilweise oder perfluoriertes C₁- bis C₆ Alkyl, C₃- bis C₇-Cycloalkyl, unsubstituiertes oder substituiertes Phenyl. In R' ist C₃- bis C₇-Cycloalkyl beispielweise Cyclopropyl, Cyclobutyl, Cyclopentyl, Cyclohexyl oder Cycloheptyl.

In R' bedeutet substituiertes Phenyl, durch C₁- bis C₆-Alkyl, C₁- bis C₆-Alkenyl, NO₂, F, Cl, Br, I, C₁-C₆-Alkoxy, SCF₃, SO₂CF₃, SO₂CH₃, COOR", SO₂X', SO₂NR"₂ oder SO₃R" substituiertes Phenyl, wobei X' F, Cl oder Br und R" ein nicht oder teilweise fluoriertes C₁- bis C₆-Alkyl oder C₃- bis C₇-Cycloalkyl wie für R' definiert bedeutet, beispielsweise, o-, m- oder p-Methylphenyl, o-, m- oder p-Ethylphenyl, o-, m- oder p-Propylphenyl, o-, m- oder p-Isopropylphenyl, o-, m- oder p-Nitrophenyl, o-, m- oder p-Methoxyphenyl, o-, m- oder p-Ethoxyphenyl, o-, m-, p-(Trifluormethyl)phenyl, o-, m-, p-(Trifluormethoxy)phenyl, o-, m-, p-(Trifluormethylsulfonyl)phenyl, o-, m- oder p-Fluorphenyl, o-, m- oder p-Chlorphenyl, o-, m- oder p-Bromphenyl, o-, m- oder p-Iodphenyl, weiter bevorzugt 2,3-, 2,4-, 2,5-, 2,6-, 3,4- oder 3,5-Dimethylphenyl, 2,3-, 2,4-, 2,5-2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2,3-, 2,4-, 2,5-, 2,6-, 3,4- oder 3,5-Dimethoxyphenyl, 5-Fluor-2-methylphenyl, 3,4,5-Trimethoxyphenyl oder 2,4,5-Trimethylphenyl.

In den Substituenten R, R¹ bis R⁷ oder R^{1'} bis R^{4'} können auch ein oder zwei nicht benachbarte, nicht direkt am Heteroatom gebundene und nicht ω-ständige Kohlenstoffatome, durch Atome und/oder Atomgruppierungen ausgewählt aus der Gruppe -O-, -C(O)- -S-, -S(O)- oder -SO₂- ersetzt werden.

Ohne Einschränkung der Allgemeinheit sind Beispiele für derart modifizierte Substituenten R, R¹ bis R⁷ und R^{1'} bis R^{4'}:
- OCH₃, -OCH(CH₃)₂, -CH₂OCH₃, -CH₂-CH₂-O-CH₃, -C₂H₄OCH(CH₃)₂, C₂H₄SC₂H₅, -C₂H₄SCH(CH₃)₂, -S(O)CH₃, -SO₂CH₃, -SO₂C₆H₅, -SO₂C₃H₇, -SO₂CH(CH₃)₂, -SO₂CH₂CF₃, -CH₂SO₂CH₃, -O-C₄H₈-O-C₄H₉, -CF₃, -C₂F₅, -C₃F₇, -C₄F₉, -CF₂CF₂H, -CF₂CHFCF₃, -CF₂CH(CF₃)₂, -C₂F₄N(C₂F₅)C₂F₅, -CHF₂, -CH₂CF₃, -C₂F₂H₃, -C₃FH₆, -CH₂C₃F₇, -CH₂C(O)OCH₃, -CH₂C(O)CH₃, -CH₂C₆H₅ oder -C(O)C₆H₅.

Die Substituenten R¹ bis R⁷ sind jeweils unabhängig voneinander bevorzugt eine geradkettige oder verzweigte Alkylgruppe mit 1 bis 10 C-Atomen. Die Substituenten R¹ und R², R³ und R⁴ und R⁵ und R⁶ in Verbindungen der Formeln (2) und (3) können dabei gleich oder verschieden sein. Besonders bevorzugt sind R¹ bis R⁷ jeweils unabhängig voneinander Methyl, Ethyl, n-Propyl, Isopropyl, n-Butyl, sek.-Butyl, Phenyl oder Cyclohexyl, ganz besonders bevorzugt Methyl, Ethyl, n-Propyl, Isopropyl oder n-Butyl. Die Substituenten R und R¹ bis R⁷ können jedoch ebenfalls durch weitere funktionelle Gruppen substituiert sein, beispielsweise durch CN, SO₂R', SO₂OR', SO₂NH₂, SO₂NR'₂, C(O)NH₂, C(O)NR'₂ oder C(O)OR'. R' hat dabei eine zuvor definierte Bedeutung.

Als Substituenten R^{1'} bis R^{4'} von Verbindungen der Formel (4) kommen erfindungsgemäß dabei neben Wasserstoff bevorzugt in Frage: CN, C₁- bis C₂₀-, insbesondere C₁- bis C₁₂-Alkylgruppen, und gesättigte oder ungesättigte, d.h. auch aromatische, C₃- bis C₇-Cycloalkylgruppen, die mit C₁- bis C₆-Alkylgruppen substituiert sein können, insbesondere Phenyl oder Aryl-C₁-C₆-alkyl.

Die Substituenten R^{1'} und R^{4'} sind jeweils unabhängig voneinander insbesondere bevorzugt CN, Methyl, Ethyl, Isopropyl, Propyl, Butyl, sek.-Butyl, Pentyl, Hexyl, Octyl, Decyl, Cyclohexyl, Phenyl, Phenylpropyl oder Benzyl. Sie sind ganz besonders bevorzugt Methyl, CN, Ethyl, n-Butyl oder Hexyl. In Pyrrolidinium- oder Piperidinium-Verbindungen sind die beiden Substituenten R^{1'} und R^{4'} bevorzugt unterschiedlich.

Der Substituent R^{2'} oder R^{3'} ist jeweils unabhängig voneinander insbesondere Wasserstoff, Dimethylamino, Diethylamino, Methyl, Ethyl, Isopropyl, Propyl, Butyl, sek.-Butyl, Cyclohexyl, Phenyl oder Benzyl. Besonders bevorzugt ist R^{2'} Wasserstoff, Methyl, Ethyl, Isopropyl, Propyl, Butyl, sek.-Butyl oder Dimethylamino. Ganz besonders bevorzugt sind R^{2'} und R^{3'} Wasserstoff.

In einer Ausführungsform sind die Alkylgruppen als Substituenten R und R¹ bis R⁶ sowie R^{1'} und R^{4'} der heterocyclischen Kationen der Formel (4) unterschiedlich von der Alkylgruppe des Anions im Onium-Alkylsulfit.

Das erfindungsgemäß hergestellte Onium-Alkylsulfit kann jedoch auch Alkylgruppen im Kation haben, die zu der Alkylgruppe im Anion gleich sind, die jedoch erfindungsgemäß nicht durch Alkylierung eingeführt wurden.

Bis zu vier Substituenten des Guanidinium-Kations [C(NR¹R²)(NR³R⁴)(NR⁵R⁶)]⁺ können auch paarweise derart verbunden sein, dass mono-, bi- oder polycyclische Kationen entstehen.

Ohne Einschränkung der Allgemeinheit sind Beispiele für solche Guanidinium-Kationen: 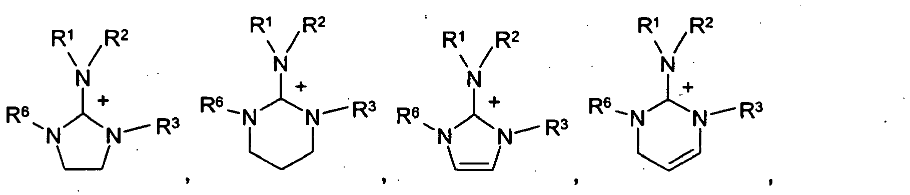 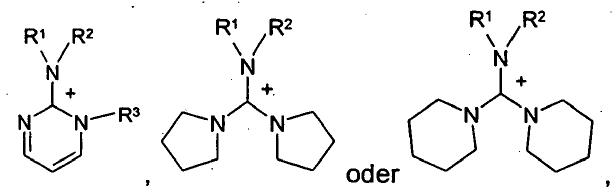 wobei die Substituenten R¹ bis R³ und R⁶ eine zuvor angegebene oder besonders bevorzugte Bedeutung haben können.

Gegebenenfalls können die Carbocyclen oder Heterocyclen der zuvor angegebenen Guanidinium-Kationen noch durch C₁- bis C₆-Alkyl, C₁- bis C₆-Alkenyl, NO₂, F, Cl, Br, I, C₁-C₆-Alkoxy, SCF₃, SO₂CF₃, SO₂CH₃, CN, COOR", SO₂NR"₂, SO₂X' oder SO₃R" substituiert sein, wobei X' F, Cl oder Br und R" ein nicht oder teilweise fluoriertes C₁- bis C₆-Alkyl oder C₃- bis C₇-Cycloalkyl wie für R' definiert bedeutet oder durch substituiertes oder unsubstituiertes Phenyl substituiert sein.

Bis zu vier Substituenten des Thiouroniumkations [(R¹R²N)-C(=SR⁷)(NR³R⁴)]⁺ können auch paarweise derart verbunden sein, dass mono-, bi- oder polycyclische Kationen entstehen.

Ohne Einschränkung der Allgemeinheit sind Beispiele für solche Kationen im folgenden angegeben: 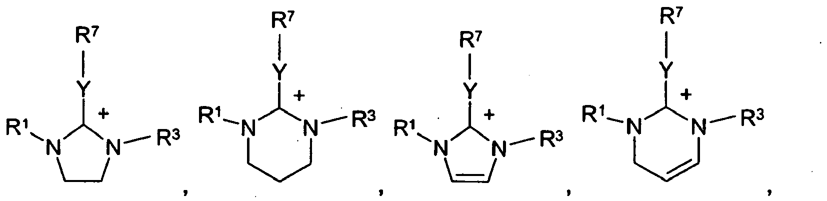 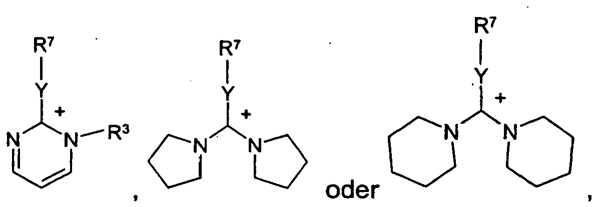 wobei Y = S und die Substituenten R¹, R³ und R⁷ eine zuvor angegebene oder besonders bevorzugte Bedeutung haben können.

Gegebenenfalls können die Carbocyclen oder Heterocyclen der zuvor angegebenen Thiouronium-Kationen noch durch C₁- bis C₆-Alkyl, C₁- bis C₆-Alkenyl, NO₂, F, Cl, Br, I, C₁-C₆-Alkoxy, SCF₃, SO₂CF₃, SO₂CH₃, COOR", SO₂NR"₂, SO₂X' oder SO₃R" substituiert sein, wobei X' F, Cl oder Br und R" ein nicht, teilweise oder perfluoriertes C₁- bis C₆-Alkyl oder C₃- bis C₇-Cycloalkyl wie für R' definiert bedeutet oder durch substituiertes oder unsubstituiertes Phenyl substituiert sein.

Die Substituenten R^{1'} bis R^{4'} können jedoch ebenfalls durch weitere funktionelle Gruppen substituiert sein, beispielsweise durch CN, SO₂R', SO₂OR' oder COOR'. R' hat dabei eine zuvor definierte Bedeutung.

HetN⁺ der Formel (4) ist bevorzugt 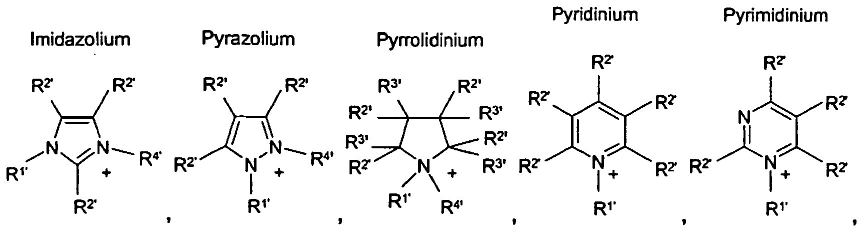 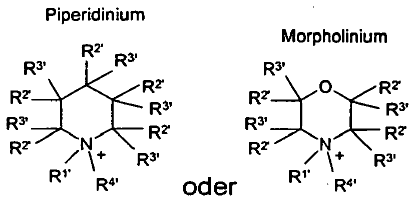 wobei die Substituenten R^{1'} bis R^{4'} jeweils unabhängig voneinander eine zuvor beschriebene Bedeutung haben.

HetN⁺ ist besonders bevorzugt Imidazolium, Pyrrolidinium oder Pyridinium, wie zuvor definiert, wobei die Substituenten R^{1'} bis R^{4'} jeweils unabhängig voneinander eine zuvor beschriebene Bedeutung haben.

In den genannten Formeln (1) bis (4) kann erfindungsgemäß Hal durch Carboxylate ersetzt sein, wie vorab definiert. Die Wahl des Anions schränkt die Auswahl des Kations nicht ein.

Als symmetrisch substituiertes Dialkylsulfit in den erfindungsgemäßen Verfahren wird bevorzugt ein Dialkylsulfit mit einer geradkettigen oder verzweigten Alkylgruppe mit 1-10 C-Atomen, bevorzugt mit 1-4 C-Atomen, besonders bevorzugt mit 1-2 C-Atomen eingesetzt. Vorzugsweise ist die Alkylgruppe eine geradkettige oder verzweigte Alkylgruppe mit 1-4 C-Atomen, wie beispielsweise Methyl, Ethyl, Isopropyl, Propyl, Butyl, sek.-Butyl oder tert.-Butyl. Bevorzugt ist die Alkylgruppe Methyl oder Ethyl. Beispiele für symmetrisch substituierte Dialkylsulfite sind Dimethylsulfit, Diethylsulfit, Di-(n-propyl)sulfit, Di-(iso-propyl)sulfit, Di-(n-butyl)sulfit oder Di-(sek-butyl)sulfit. Vorzugsweise wird Dimethylsulfit oder Diethylsulfit eingesetzt.

Die eingesetzten symmetrischen Dialkylsulfite sind in der Regel kommerziell erhältlich oder können nach Syntheseverfahren hergestellt werden, wie sie aus der Literatur, z.B. in den Standardwerken wie Houben-Weyl, Methoden der organischen Chemie, Georg-Thieme-Verlag, Stuttgart oder Richard C. Larock, Comprehensive Organic Transformations, 2nd Edition, Wiley-VCH, New York, 1999, oder aus dem Artikel von W. Voss und E. Blanke, Justus Liebigs Ann. Chem., 485 (1931), S. 258-279 bekannt sind. Dabei kann man auch von an sich bekannten, hier nicht näher erwähnten Varianten Gebrauch machen.

Als unsymmetrisch substituiertes Dialkylsulfit wird bevorzugt ein Dialkylsulfit mit einer geradkettigen oder verzweigten Alkylgruppe mit 1 bis 10 C-Atomen und einer Methyl- oder Ethylgruppe als zweite Alkylgruppe, bevorzugt mit einer Alkylgruppe mit 3-8 C-Atomen, eingesetzt. Beispiele für unsymmetrisch substituierte Dialkylsulfite sind Methyl-propylsulfit, Methylbutylsulfit, Ethyl-butylsulfit, Methyl-pentylsulfit, Ethyl-pentylsulfit, Methylhexylsulfit, Ethyl-hexylsulfit, Methyl-heptylsulfit, Ethyl-heptylsulfit, Methyloctylsulfit oder Ethyl-octylsulfit.

Die eingesetzten unsymmetrischen Dialkylsulfite können nach Syntheseverfahren hergestellt werden, wie sie aus der Literatur, z.B. in den Standardwerken wie Houben-Weyl, Methoden der organischen Chemie, Georg-Thieme-Verlag, Stuttgart oder Richard C. Larock, Comprehensive Organic Transformations, 2nd Edition, Wiley-VCH, New York, 1999, oder aus dem Artikel von W. Voss und E. Blanke, Justus Liebigs Ann. Chem., 485 (1931), S. 258-279, oder von V.M. Pavlov, J. Gen. Chem. USSR (Eng. Transl.), 41 (1971), S. 2559-2561 bekannt sind. Dabei kann man auch von an sich bekannten, hier nicht näher erwähnten Varianten Gebrauch machen.

Ein allgemeines Schema fasst das erfindungsgemäße Verfahren zusammen, wobei der Pfeil bei der entstehenden Alkylhalogenverbindung ein Symbol für die Flüchtigkeit der Verbindung darstellt:

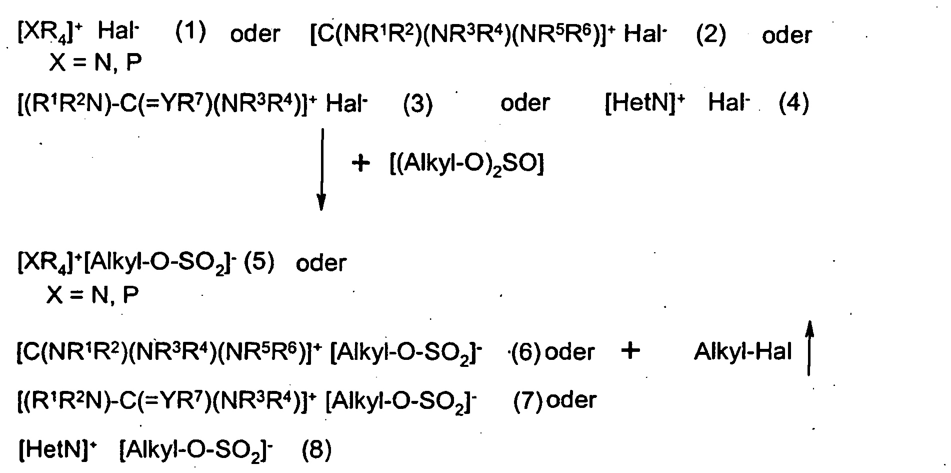

Die Substituenten R, R¹ bis R⁷ und HetN⁺ der Verbindungen der Formeln (1) bis (8) entsprechen den Bedeutungen, wie zuvor beschrieben.

Die Reaktion mit Dialkylsulfiten wird bei Temperaturen zwischen 0 und 70°C, vorzugsweise bei Temperaturen zwischen 20 und 60°C durchgeführt, und besonders bevorzugt bei Raumtemperatur. Die Auswahl der optimalen Reaktionstemperatur hängt dabei von der Höhe des Überschusses des Dialkylsulfits und von der Art des Halogenids und des eingesetzten Dialkylsulfits ab. Generell sind höhere Temperaturen und längere Reaktionszeiten bei Dialkylsulfiten mit längeren Alkylketten nötig.

Es wird kein Lösungsmittel benötigt. Es können jedoch auch Lösungsmittel eingesetzt werden, beispielsweise Dimethoxyethan, Acetonitril, Aceton, Tetrahydrofuran, Dimethylformamid, Dimethylsulfoxid, Dioxan, Propionitril, Dichlormethan oder Gemische untereinander.

Die Umsetzung wird mit einem Überschuss oder equimolarer Menge an Dialkylsulfit durchgeführt.

Alternativ lassen sich Oniumsalze mit Alkylsulfit-Anionen durch direkte Alkylierung organischer Basen mit Dialkylsulfiten herstellen. Die Versuche zur Alkylierung von, Dimethylanilin und Pyridin mit Dimethylsulfit ist im Artikel von W. Voss und E. Blanke, Justus Liebigs Ann. Chem., 485 (1931), S. 258-279 beschrieben. Die entsprechende Bildung der Methylsulfite mit geringer Ausbeute ist vermutet, aber nicht nachgewiesen. Der Nachteil dieser Methode liegt in der Bildung von Onium-Verbindungen, die sowohl im Kation als auch im Anion die gleiche Alkylgruppe aufweisen und damit die mögliche Vielfalt der Substitutionsmuster beschränken.

Ebenfalls Gegenstand der vorliegenden Erfindung sind die Onium-Alkylsulfite 1-Butyl-3-methylimidazoliumethylsulfit und 1-Ethyl-3-methylimid-azoliumethylsulfit. Durch Anwendung des erfindungsgemäßen Verfahrens wird der Zugang zu diesen Verbindungen vereinfacht und damit die Möglichkeit der Nutzung dieser Verbindungen vergrößert.

Darüber hinaus sind Onium-Alkylsulfite der Formel (9),

[Kt]⁺ [Alkyl-O-SO₂]⁻ (9)

wobei
Kt unsymmetrische Tetraalkylammonium-, symmetrische und unsymmetrische Phosphonium-, Guanidinium- Thiouronium-Kationen oder heterocyclische Kationen bedeutet, erhältlich nach dem erfindungsgemäßen Verfahren. Entsprechende Kationen sind bereits bei der Beschreibung der geeigneten Onium-Halogenide genannt.

Es versteht sich für den Fachmann von selbst, dass alle vorab genannten Substituenten wie beispielsweise H, N, O, Cl, F durch die entsprechenden Isotope ersetzt sein können.

Die Verwendung der Verbindungen der Formel (9) als Lösungsmittel, Lösungsmittelzusatz, Wärmeträger, Reduktionsmittel, Antioxidans, Phasentransfer-Katalysator, als Extraktionsmittel, als Additiv, als oberflächenaktive Substanz, als Elektrolyt in galvanischen Zellen, als Modifier oder als Weichmacher ist gegeben.

Die Verbindungen der Formel (9) eignen sich darüber hinaus auch als Ausgangsmaterial für die Synthese von Onium-Salzen mit Alkylsulfat-Anionen durch Oxidation. Dieses Verfahren stellt eine vorteilhafte Alternative zur Herstellung von Onium-Salzen mit Alkylsulfat-Anionen dar, die gegenüber der herkömmlichen direkten Alkylierung organischer Basen mit Dialkylsulfaten, z.B. Dimethylsulfat, den Vorteil haben, dass auf den Einsatz des hochgiftigen Dialkylsulfats verzichtet werden kann. Als Oxidationsmittel bei dieser Reaktion eignen sich alle dem Fachmann bekannten Oxidationsmittel, z.B. Sauerstoff.

Im Falle des Einsatzes als Lösungsmittel oder Lösungsmittelzusatzes eignen sich die Verbindungen der Formel (8) in jeder dem Fachmann bekannten Art von Reaktion, z.B. Übergangsmetall-, Enzym-, oder mit anderen Biokatalysatoren katalysierte Reaktionen, wie z.B. Hydroformilierungsreaktionen, Oligomerisierungsreaktionen, C-C-Bindungsknüpfungsreaktionen, z.B. die Heck-Kuppfung, aber auch Veresterungen, Isomerisierungsreaktionen oder Reaktionen zur Amidbindungsknüpfung.

Auch ohne weitere Ausführungen wird davon ausgegangen, dass ein Fachmann die obige Beschreibung im weitesten Umfang nutzen kann. Die bevorzugten Ausführungsformen und Beispiele sind deswegen lediglich als beschreibende, keineswegs als in irgendeiner Weise limitierende Offenbarung aufzufassen.

Die NMR-Spektren wurden an Lösungen in deuterierten Lösungsmitteln bei 20°C an einem Bruker ARX 400 Spektrometer mit einem 5 mm Breitbandkopf ¹H/BB mit Deuterium Lock gemessen, falls nicht in den Beispielen angegeben. Die Messfrequenzen der verschiedenen Kerne sind: ¹H: 400,13 MHz und ³¹P .161.98 MHz. Die Methode der Referenzierung wird bei jedem Spektrum bzw. bei jedem Datensatz separat angegeben.

### Beispiele:

### Beispiel 1: 1-Butyl-3-methylimidazoliummethylsulfit

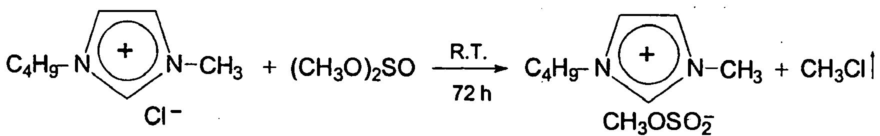

Eine Mischung aus 8,81 g (50,4 mmol) 1-Butyl-3-methylimidazoliumchlorid und 5,56 g (50,5 mmol) Dimethylsulfit wird bei Raumtemperatur für 72 Stunden unter Inertgasatmosphäre (Stickstoff) im geschlossenem 100 ml Reaktionsgefäß gerührt. Das Ende der Reaktion wird durch NMR-Messung bestimmt. Das Produkt wird im Vakuum bei 13.3 Pa und 60°C (Temperatur des Ölbades) innerhalb von 1 Stunde abgepumpt. Man erhält 11.8 g flüssiges 1-Butyl-3-methylimidazoliummethylsulfit. Die Ausbeute ist nahezu quantitativ. Das Produkt wird mittels NMR-Spektroskopie untersucht.

¹H NMR (Referenz: TMS; Lösungsmittel: CD₃CN), ppm: 0.88 t (CH₃); 1.28 m (CH₂); 1.79 m (CH₂); 3.17 s (CH₃O); 3.86 s (CH₃); 4.19 t (CH₂); 7.54 d,d (CH); 7.56 d,d (CH); 9.66 br. s. (CH); ³J_{H,H} = 7.1 Hz; J_{H,H}= 1.5 Hz.

### Beispiel 2: 1-Butyl-3-methylimidazoliumethylsulfit

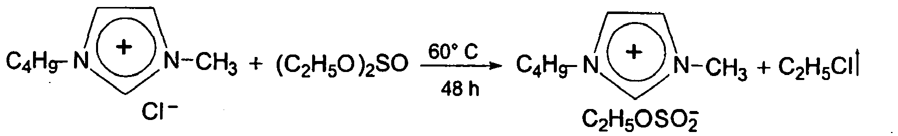

Eine Mischung aus 6,38 g (36,5 mmol) 1-Butyl-3-methylimidazoliumchlorid und 6,66 g (48,2 mmol) Diethylsulfit wird bei 60-70°C (Temperatur des Ölbades) für 48 Stunden unter Inertgasatmosphäre (Stickstoff) im geschlossenem Reaktionsgefäß mit Druckventile für 1-1.5 bar Überdruck gerührt. Das Ende der Reaktion wird durch NMR-Messung bestimmt. Das Produkt wird im Vakuum bei 13.3 Pa und 60°C (Temperatur des Ölbades) innerhalb von 2 Stunden abgepumpt. Man erhält 8.82 g flüssiges 1-Butyl-3-methylimidazoliumethylsulfit. Die Ausbeute ist nahezu quantitativ. Das Produkt wird mittels NMR-Spektroskopie untersucht.

¹H NMR (Referenz: TMS; Lösungsmittel: CD₃CN), ppm: 0.89 t (CH₃); 1.06 t (CH₃); 1.28 m (CH₂); 1.79 m (CH₂); 3.56 q (CH₂O); 3.87 s (CH₃); 4.18 t (CH₂); 7.47 m (CH); 7.51 m (CH); 9.56 br. s. (CH); ³J_{H,H} = 7.1 Hz.

### Beispiel 3: 1-Butyl-3-methylimidazoliumethylsulfit

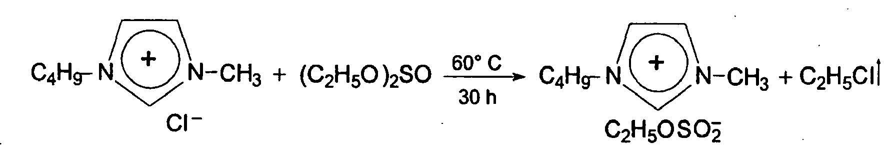

Eine Mischung aus 3,14 g (18,0 mmol) 1-Butyl-3-methylimidazoliumchlorid und 4,97 g (36,0 mmol) Diethylsulfit wird bei 60-70°C (Temperatur des Ölbades) für 30 Stunden unter Inertgasatmosphäre (Stickstoff) im geschlossenem Reaktionsgefäß mit Druckventile für 1-1.5 bar Überdruck gerührt. Das Ende der Reaktion wird durch NMR-Messung bestimmt. Das Produkt wird im Vakuum bei 13.3 Pa und 60°C (Temperatur des Ölbades) innerhalb von 14 Stunden abgepumpt. Man erhält 4.41 g flüssiges 1-Butyl-3-methylimidazoliumethylsulfit. Die Ausbeute ist nahezu quantitativ. Das Produkt wird mittels NMR-Spektroskopie untersucht.

¹H NMR (Referenz: TMS; Lösungsmittel: CD₃CN), ppm: 0.89 t (CH₃); 1.06 t (CH₃); 1.28 m (CH₂); 1.79 m (CH₂): 3.56 q (CH₂O); 3.87 s (CH₃); 4.18 t (CH₂); 7.47 m (CH); 7.51 m (CH); 9.56 br. s. (CH); ³J_{H,H} = 7.1 Hz.

### Beispiel 4:1-Ethyl-3-methylimidazoliumethylsulfit

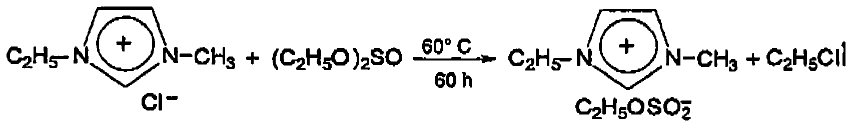

Eine Mischung aus 4,90 g (33,4 mmol) 1-Ethyl-3-methylimidazoliumchlorid und 5,67 g (41,0 mmol) Diethylsulfit wird bei 60°C (Temperatur des Ölbades) für 60 Stunden unter Inertgasatmosphäre (Stickstoff) im geschlossenem Reaktionsgefäß mit Druckventile für 1-1.5 bar Überdruck gerührt. Das Ende der Reaktion wird durch NMR-Messung bestimmt. Das Produkt wird im Vakuum bei 13.3 Pa und 60°C (Temperatur des Ölbades) innerhalb von 2 Stunden abgepumpt. Man erhält 7.23 g flüssiges 1-Ethyl-3-methylimidazoliumethylsulfit. Die Ausbeute ist nahezu quantitativ. Das Produkt wird mittels NMR-Spektroskopie untersucht.

¹H NMR (Referenz: TMS; Lösungsmittel: CD₃CN), ppm: 1.06 t (CH₃); 1.43 t (CH₃); 3.56 q (CH₂O); 3.87 s (CH₃); 4.22 q (CH₂); 7.47 m (CH); 7.53 m (CH); 9.49 br. s. (CH); ³J_{H,H} = 7.3 Hz.

### Beispiel 5 : Tetrabutylammoniummethylsulfit

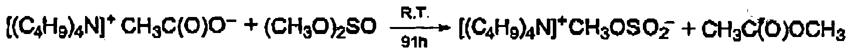

Eine Mischung aus 1.84 g (6.1 mmol) Tetrabutylammoniumacetat und 0.672 g (6.1 mmol) Dimethylsulfit wird bei Raumtemperatur für 91 Stunden unter Inertgasatmosphäre (Stickstoff) im geschlossenem Reaktionsgefäß gerührt. Das Ende der Reaktion wird durch NMR-Messung bestimmt. Das Produkt wird im Vakuum bei 13.3 Pa bei Raumtemperatur innerhalb von 2 Stunden abgepumpt. Man erhält 1.98 g flüssiges Tetrabutylammoniummethylsulfit. Die. Ausbeute ist nahezu quantitativ. Das Produkt wird mittels NMR-Spektroskopie untersucht.

¹H NMR (Referenz: TMS; Lösungsmittel: CD₃CN), ppm: 0.95 t (4CH₃); 1.34 t,q (4CH₂); 1.60 m (4CH₂); 3.10 m (4CH₂); 3.15 s (CH₃O); ³J_{H,H} = 7.4 Hz.

### Beispiel 6 : Tetrabutylammoniumethylsulfit

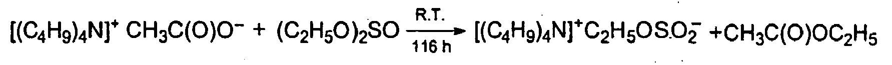

Eine Mischung aus 3.69 g (12.2 mmol) Tetrabutylammoniumacetat und 0.672 g (12.2 mmol) Dimethylsulfit wird bei Raumtemperatur für 116 Stunden unter Inertgasatmosphäre (Stickstoff) im geschlossenem Reaktionsgefäß gerührt. Das Ende der Reaktion wird durch NMR-Messung bestimmt. Das Produkt wird im Vakuum bei 13.3 Pa bei Raumtemperatur innerhalb von 3 Stunden abgepumpt. Man erhält 4.19 g flüssiges Tetrabutylammoniumethylsulfit. Die Ausbeute ist nahezu quantitativ. Das Produkt wird mittels NMR-Spektroskopie untersucht.

¹H NMR (Referenz: TMS; Lösungsmittel: CD₃CN), ppm: 0.90 t (4CH₃); 1.06 t (CH₃); 1.33 t,q (4CH₂); 1.60 m (4CH₂); 3.13 m (4CH₂); 3.52 q (CH₂O); ³J_{H},_{H} = 7.4 Hz.

### Beispiel 7: Tetramethylammoniumethylsulfit

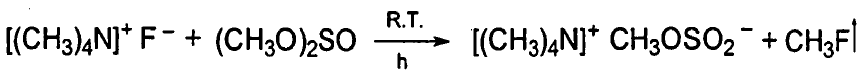

Eine Mischung aus 1.34 g (14.4 mmol) Tetramethylammoniumfluorid und 3.17 g (28.8 mmol) Dimethylsulfit wird bei 70°C (Temperatur des Ölbades) für 2 Stunden unter Inertgasatmosphäre (Stickstoff) im geschlossenem Reaktionsgefäß mit Druckventile für 1-1.5 bar Überdruck gerührt. Das Ende der Reaktion wird durch NMR-Messung bestimmt. Der Überschuss von Dimethylsulfit wird im Vakuum bei 13.3 Pa und 100°C (Temperatur des Ölbades) innerhalb von 2 Stunden abgepumpt. Man erhält 2.34 g festes Tetrabutylammoniumethylsulfit. Der Schmelzpunkt beträgt 155-157°C. Die Ausbeute beträgt 96.3 %. Das Produkt wird mittels NMR-Spektroskopie untersucht.

¹H NMR (Referenz: TMS; Lösungsmittel: D₂O), ppm: 3.05 s (4CH₃); 3.21 s (CH₃O).

### Beispiel 8: 1,3-Dimethylimidazoliummethylsulfit

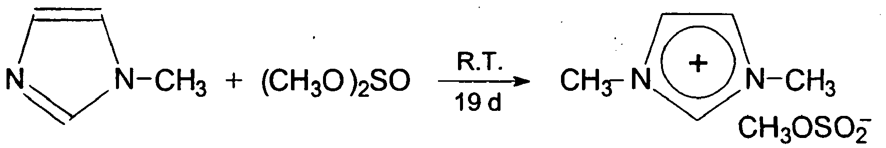

Eine Mischung aus 5.89 g (71.7 mmol) N-Methylimidazol und 7.90 g (71.7 mmol) Dimethylsulfit wird bei Raumtemperatur für 19 Tage unter Inertgasatmosphäre (Stickstoff) in einer geschlossenen Rundflasche (50 ml) gerührt. Das Ende der Reaktion wird durch NMR-Messung bestimmt. Das Produkt wird im Vakuum 13.3 Pa bei Raumtemperatur innerhalb von 12 Stunden abgepumpt. Man erhält 13.63 g flüssiges 1,3-Dimethylimidazoliummethylsulfit. Die Ausbeute ist nahezu quantitativ. Das Produkt wird mittels NMR-Spektroskopie untersucht.

¹H NMR (Referenz: TMS; Lösungsmittel: CD₃CN), ppm: 3.18 s (CH₃O); 3.86 s (2CH₃); 7.51 d (2CH); 9.45 br. s. (CH); ⁴J_{H,H} = 1.6 Hz.

### Beispiel 9: Oxidation von 1,3-Dimethylimidazoliummethylsulfit

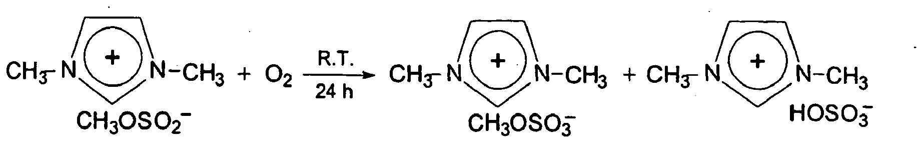

Gasförmiger Sauerstoff wird durch eine Lösung aus 1.33 g 1,3-Dimethylimidazoliummethylsulfit in 20 ml Acetonitril für 24 Stunden durchgeblasen. Das feste Produkt wird abfiltriert und im Vakuum 13.3 Pa bei 50° C innerhalb von 1 Stunde abgepumpt. Man erhält 0.71 g festen Produkts, das mittels NMR und Elementaranalyse als 1,3-Dimethylimidazoliumhydrogensulfat nachgewiesen werden kann.

¹³C NMR (Referenz: TMS-external; Lösungsmittel: D₂O), ppm: 36.4 q (2CH₃), 124.0 d (2CH), 137.2 d (CH), ¹J_{C,H} = 144 Hz, ¹J_{C,H} = 202 Hz, ¹J_{C,H} = 223 Hz

Elementaranalyse: gefunden, %: C 32.08; H 5.12; N 14.91; S 17.01; berechnet für C₅H₁₀N₂O₄S, %: C 30.93; H 5.19; N 14.42; S 16.51.

Acetonitril wird mittels Rotationsverdampfer entfernt und das verbliebene Produkt (0.5 g) wird mittels NMR-Spektroskopie untersucht. Das Spektrum ist identisch zum Spektrum von 1,3-Dimethyl-imidazoliummethylsulfat.

¹H NMR (Referenz: TMS; Lösungsmittel: CD₃CN), ppm: 3.50 s (CH₃O); 3.84 s (2CH₃); 7.39 d (2CH); 8.86 br. s. (CH); ⁴J_{H,H} = 1.5 Hz.

## Patentansprüche

1. Verfahren zur Herstellung von Onium-Alkylsulfiten durch Umsetzung eines Onium-Halogenids oder -Carboxylats mit einem symmetrisch substituierten Dialkylsulfit oder mit einem unsymmetrisch substituierten Dialkylsulfit bei Temperaturen von 0 bis 70°C.

2. Verfahren nach Anspruch 1, **dadurch gekennzeichnet, dass** die Umsetzung mit einem symmetrisch substituierten Dialkylsulfit, wobei die Alkylgruppe 1 bis 10 C-Atome haben kann, erfolgt.

3. Verfahren nach Anspruch 1, **dadurch gekennzeichnet, dass** die Umsetzung mit einem unsymmetrisch substituierten Dialkylsulfit, wobei eine Alkylgruppe 1 bis 10 C-Atome haben kann und die zweite Alkylgruppe Methyl oder Ethyl bedeutet, erfolgt.

4. Verfahren nach einem oder mehreren der Ansprüche 1 bis 3, **dadurch gekennzeichnet, dass** das Halogenid ein Ammoniumhalogenid, Phosphoniumhalogenid, Thiouroniumhalogenid, Guanidiniumhalogenid oder Halogenid mit heterocyclischem Kation ist, wobei die Halogenide aus der Gruppe Fluoride, Chloride oder Bromide ausgewählt werden können.

5. Verfahren nach einem oder mehreren der Ansprüche 1 bis 4, **dadurch gekennzeichnet, dass** das Carboxylate ein Ammoniumacetat, Phosphoniumacetat, ein Guanidiniumacetat oder ein Acetat mit einem heterocyclischem Kation ist.

6. Verfahren nach einem oder mehreren der Ansprüche 1 bis 4, **dadurch gekennzeichnet, dass** das Halogenid der Formel (1) entspricht,
[XR₄]⁺ Hal⁻ (1),
wobei
X N, P
Hal F, Cl oder Br und
R jeweils unabhängig voneinander
H, wobei nicht alle Substituenten R gleichzeitig H sein dürfen, geradkettiges oder verzweigtes Alkyl mit 1-20 C-Atomen, geradkettiges oder verzweigtes Alkenyl mit 2-20 C-Atomen und einer oder mehreren Doppelbindungen bedeutet,
geradkettiges oder verzweigtes Alkinyl mit 2-20 C-Atomen und einer oder mehreren Dreifachbindungen,
gesättigtes, teilweise oder vollständig ungesättigtes Cycloalkyl mit 3-7 C-Atomen, das mit Alkylgruppen mit 1-6 C-Atomen substituiert sein kann bedeutet,
wobei ein oder mehrere R teilweise mit Cl, Br und/oder CN oder teilweise oder vollständig mit -F oder F und Cl, oder F und Br, oder F, Cl und Br substituiert sein können, wobei jedoch nicht alle vier oder drei R vollständig mit Halogenen substituiert sein dürfen,
und wobei ein oder zwei nicht benachbarte und nicht α- oder ω-ständige Kohlenstoffatome des R, durch Atome und/oder Atomgruppierungen ausgewählt aus der Gruppe -O-, -C(O)-, -S-, -S(O)- oder -SO₂- ersetzt sein können.

7. Verfahren nach einem oder mehreren der Ansprüche 1 bis 4, **dadurch gekennzeichnet, dass** das Halogenid der Formel (2) entspricht,
[C(NR¹R²)(NR³R⁴)(NR⁵R⁶)]⁺ Hal⁻ (2),
wobei
Hal F, Cl oder Br und
R¹ bis R⁶ jeweils unabhängig voneinander
Wasserstoff oder CN,
geradkettiges oder verzweigtes Alkyl mit 1 bis 20 C-Atomen, geradkettiges oder verzweigtes Alkenyl mit 2-20 C-Atomen und einer oder mehreren Doppelbindungen,
geradkettiges oder verzweigtes Alkinyl mit 2-20 C-Atomen und einer oder mehreren Dreifachbindungen,
gesättigtes, teilweise oder vollständig ungesättigtes Cycloalkyl mit 3-7 C-Atomen, das mit Alkylgruppen mit 1-6 C-Atomen substituiert sein kann, bedeutet,
wobei ein oder mehrere der Substituenten R¹ bis R⁶ teilweise mit -NO₂, CN, Cl und/oder Br, teilweise oder vollständig mit F, oder F und Cl, oder F und Br oder F, Cl und Br substituiert sein können, wobei jedoch nicht alle Substituenten an einem N-Atom vollständig mit Halogenen substituiert sein dürfen,
wobei die Substituenten R¹ bis R⁶ paarweise durch Einfach- oder Doppelbindung miteinander verbunden sein können und
wobei ein oder zwei nicht benachbarte, nicht direkt am Heteroatom gebundene und nicht ω-ständige Kohlenstoffatome der Substituenten R¹ bis R⁶ durch Atome und/oder Atomgruppierungen ausgewählt aus der Gruppe -O-, -C(O)-, -S-, -S(O)- oder -SO₂- ersetzt sein können.

8. Verfahren nach einem oder mehreren der Ansprüche 1 bis 4, **dadurch gekennzeichnet, dass** das Halogenid der Formel (3) entspricht,
[(R¹R²N)-C(=SR⁷)(NR³R⁴)]⁺ Hal⁻ (3)
beschrieben werden,
wobei
Hal Cl oder Br und
R¹ bis R⁴ und R⁷ jeweils unabhängig voneinander Wasserstoff oder CN, wobei Wasserstoff für R⁷ ausgeschlossen wird, geradkettiges oder verzweigtes Alkyl mit 1 bis 20 C-Atomen, geradkettiges oder verzweigtes Alkenyl mit 2-20 C-Atomen und einer oder mehreren Doppelbindungen,
geradkettiges oder verzweigtes Alkinyl mit 2-20 C-Atomen und einer oder mehreren Dreifachbindungen,
gesättigtes, teilweise oder vollständig ungesättigtes Cycloalkyl mit 3-7 C-Atomen, das mit Alkylgruppen mit 1-6 C-Atomen substituiert sein kann, bedeutet,
wobei ein oder mehrere der Substituenten R¹ bis R⁴ und R⁷ teilweise oder vollständig mit F, Cl und/oder Br, insbesondere -F und/oder -Cl, oder teilweise mit CN substituiert sein können, wobei jedoch nicht alle Substituenten an einem N-Atom vollständig mit Halogenen substituiert sein dürfen,
wobei die Substituenten R¹ bis und R⁷ paarweise durch Einfach- oder Doppelbindung miteinander verbunden sein können und wobei ein oder zwei nicht benachbarte, nicht direkt am Heteroatom gebundene und nicht ω-ständige Kohlenstoffatome der Substituenten R¹ bis und R⁷ durch Atome und/oder Atomgruppierungen ausgewählt aus der Gruppe - O-, -C(O)-, -S-, -S(O)- oder -SO₂- ersetzt sein können.

9. Verfahren nach einem oder mehreren der Ansprüche 1 bis 4, **dadurch gekennzeichnet, dass** das Halogenid der Formel (4) entspricht,
[HetN]⁺ + Hal⁻ (4),
wobei
Hal Cl oder Br und
HetN⁺ ein heterocyclisches Kation, ausgewählt aus der Gruppe 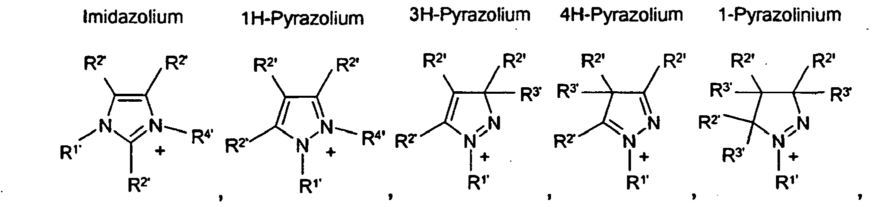 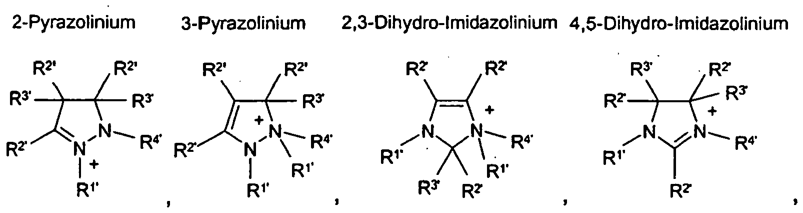 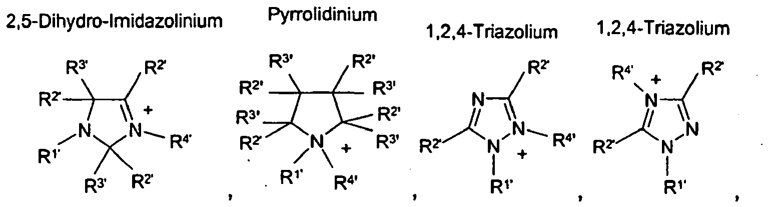 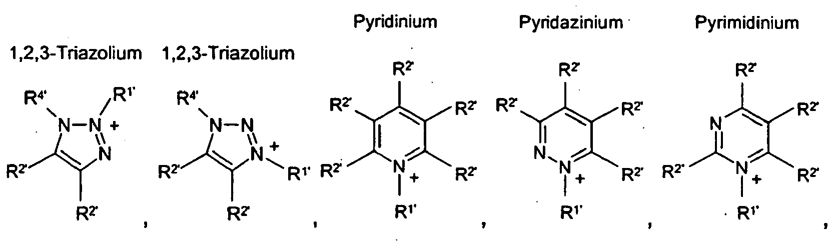 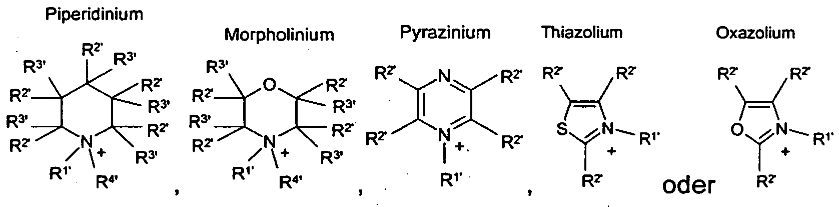 bedeutet, wobei die Substituenten
R¹, bis R⁴' jeweils unabhängig voneinander
Wasserstoff oder CN,
geradkettiges oder verzweigtes Alkyl mit 1-20 C-Atomen,
geradkettiges oder verzweigtes Alkenyl mit 2-20 C-Atomen und einer oder mehreren Doppelbindungen,
geradkettiges oder verzweigtes Alkinyl mit 2-20 C-Atomen und einer oder mehreren Dreifachbindungen,
Dialkylamino mit Alkylgruppen mit 1-4 C-Atomen, welches jedoch nicht am Heteroatom des Heterocyclus gebunden vorliegt, gesättigtes, teilweise oder vollständig ungesättigtes Cycloalkyl mit 3-7 C-Atomen, das mit Alkylgruppen mit 1-6 C-Atomen substituiert sein kann oder Aryl-C₁-C₆-alkyl bedeutet,
wobei die Substituenten R¹' und R⁴' teilweise oder vollständig mit F, Cl und/oder Br, insbesondere -F und/oder -Cl substituiert sein können, wobei jedoch nicht gleichzeitig R^{1'} und R^{4'} CN sind oder vollständig mit F oder anderen Halogenen substituiert sein dürfen,
wobei die Substituenten R^{2'} und R^{3'} teilweise oder vollständig mit F, Cl und/oder Br, insbesondere -F und/oder -Cl, oder teilweise mit NO₂ oder CN substituiert sein dürfen
und wobei ein oder zwei nicht benachbarte, nicht direkt am Heteroatom gebundene und nicht ω-ständige Kohlenstoffatome der Substituenten R^{1'} bis R^{4'}, durch Atome und/oder Atomgruppierungen ausgewählt aus der Gruppe -O-, -C(O)-, -S-, -S(O)- oder -SO₂- ersetzt sein können.

10. Verfahren nach einem oder mehreren der Ansprüche 1 bis 19, **dadurch gekennzeichnet, dass** die Reaktion des Halogenids mit dem Dialkylsulfit ohne Lösungsmittel durchgeführt wird.

11. Verwendung von Onium-Alkylsulfiten der Formel (9), erhältlich nach dem Verfahren gemäß Anspruch 1
[Kt]⁺ [Alkyl-O-SO₂]⁻ (9)
wobei Kt unsymmetrische Tetraalkylammonium-, symmetrische und unsymmetrische Phosphonium-, Guanidinium-, Thiouronium-Kationen oder heterocyclische Kationen bedeutet, als Ausgangsmaterial für die Synthese von Onium-Salzen mit Alkylsulfat-Anionen durch Oxidation.

12. 1-Butyl-3-methyl-imidazoliumethylsulfit,
1-Ethyl-3-methyl-imidazoliumethylsulfit.

## Claims

1. Process for the preparation of onium alkylsulfites by reaction of an onium halide or carboxylate with a symmetrically substituted dialkyl sulfite or with an asymmetrically substituted dialkyl sulfite at temperatures of 0 to 70°C.

2. Process according to Claim 1, **characterised in that** the reaction is carried out with a symmetrically substituted dialkyl sulfite, where the alkyl group can have 1 to 10 C atoms.

3. Process according to Claim 1, **characterised in that** the reaction is carried out with an asymmetrically substituted dialkyl sulfite, where one alkyl group can have 1 to 10 C atoms and the second alkyl group denotes methyl or ethyl.

4. Process according to one or more of Claims 1 to 3, **characterised in that** the halide is an ammonium halide, phosphonium halide, thiouronium halide, guanidinium halide or a halide with a heterocyclic cation, where the halides can be selected from the group fluorides, chlorides or bromides.

5. Process according to one or more of Claims 1 to 4, **characterised in that** the carboxylate is an ammonium acetate, phosphonium acetate, guanidinium acetate or an acetate with a heterocyclic cation.

6. Process according to one or more of Claims 1 to 4, **characterised in that** the halide conforms to the formula (1),
[XR₄]⁺ Hal⁻ (1),
where
X denotes N, P
Hal denotes F, Cl or Br and
R in each case, independently of one another, denotes H, where all substituents R cannot simultaneously be H,
straight-chain or branched alkyl having 1-20 C atoms,
straight-chain or branched alkenyl having 2-20 C atoms and one or more double bonds,
straight-chain or branched alkynyl having 2-20 C atoms and one or more triple bonds,
saturated, partially or fully unsaturated cycloalkyl having 3-7 C atoms, which may be substituted by alkyl groups having 1-6 C atoms,
where one or more R may be partially substituted by Cl, Br and/or CN or partially or fully by F, or F and Cl, or F and Br, or F, Cl and Br, but where all four or three R cannot be fully substituted by halogens,
and where one or two non-adjacent carbon atoms of the R which are not in the α- or ω-position may be replaced by atoms and/or atom groups selected from the group -O-, -C(O)-, -S-, -S(O)- or -SO₂-.

7. Process according to one or more of Claims 1 to 4, **characterised in that** the halide conforms to the formula (2),
[C(NR¹R²)(NR³R⁴)(NR⁵R⁶)]⁺ Hal⁻ (2),
where
Hal denotes F, Cl or Br and
R¹ to R⁶ each, independently of one another, denote hydrogen or CN,
straight-chain or branched alkyl having 1 to 20 C atoms,
straight-chain or branched alkenyl having 2-20 C atoms and one or more double bonds,
straight-chain or branched alkynyl having 2-20 C atoms and one or more triple bonds,
saturated, partially or fully unsaturated cycloalkyl having 3-7 C atoms, which may be substituted by alkyl groups having 1-6 C atoms,
where one or more of the substituents R¹ to R⁶ may be partially substituted by NO₂, CN, Cl and/or Br, partially or fully by F, or F and Cl, or F and Br, or F, Cl and Br, but where all substituents on an N atom cannot be fully substituted by halogens,
where the substituents R¹ to R⁶ may be connected to one another in pairs by a single or double bond and
where, in the substituents R¹ to R⁶, one or two non-adjacent carbon atoms which are not bonded directly to the heteroatom and are not in the ω-position may be replaced by atoms and/or atom groups selected from the group -O-, -C(O)-, -S-, -S(O)- or -SO₂-.

8. Process according to one or more of Claims 1 to 4, **characterised in that** the halide conforms to the formula (3),
[(R¹R²N)-C(=SR⁷)(NR³R⁴)]⁺ Hal⁻ (3),
where
Hal denotes Cl or Br and
R¹ to R⁴ and R⁷ each, independently of one another, denote hydrogen or CN, where hydrogen is excluded for R⁷,
straight-chain or branched alkyl having 1 to 20 C atoms,
straight-chain or branched alkenyl having 2-20 C atoms and one or more double bonds,
straight-chain or branched alkynyl having 2-20 C atoms and one or more triple bonds,
saturated, partially or fully unsaturated cycloalkyl having 3-7 C atoms, which may be substituted by alkyl groups having 1-6 C atoms,
where one or more of the substituents R¹ to R⁴ and R⁷ may be partially or fully substituted by F, Cl and/or Br, in particular F and/or Cl, or partially by CN, but where all substituents on an N atom cannot be fully substituted by halogens,
where the substituents R¹ to R⁴ and R⁷ may be connected to one another in pairs by a single or double bond and where, in the substituents R¹ to R⁴ and R⁷, one or two non-adjacent carbon atoms which are not bonded directly to the heteroatom and are not in the ω-position may be replaced by atoms and/or atom groups selected from the group -O-, -C(O)-, -S-, -S(O)- or -SO₂-.

9. Process according to one or more of Claims 1 to 4, **characterised in that** the halide conforms to the formula (4),
[HetN]⁺ Hal⁻ (4),
where
Hal denotes Cl or Br and
HetN⁺ denotes a heterocyclic cation selected from the group 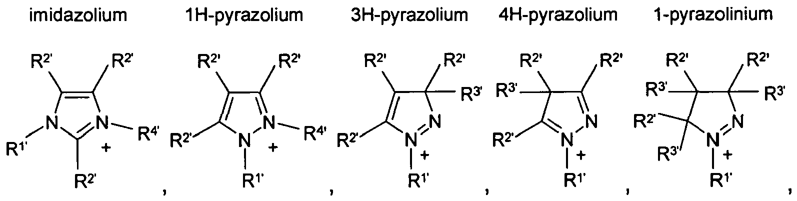 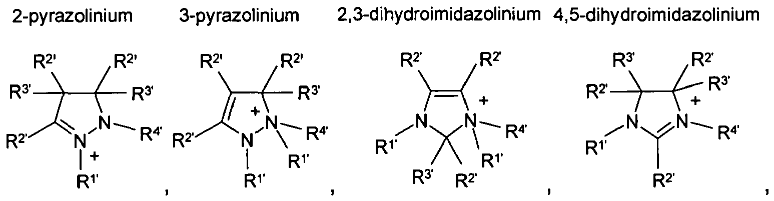 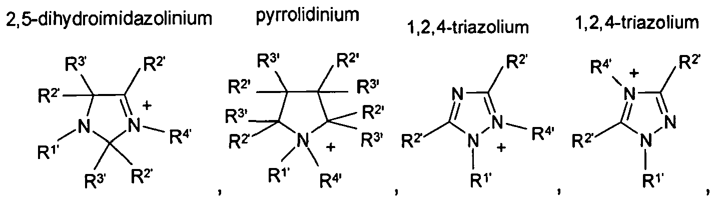 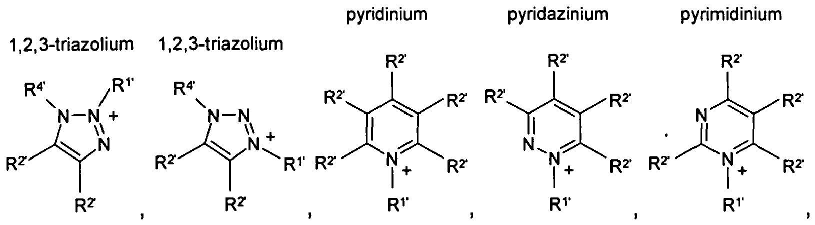 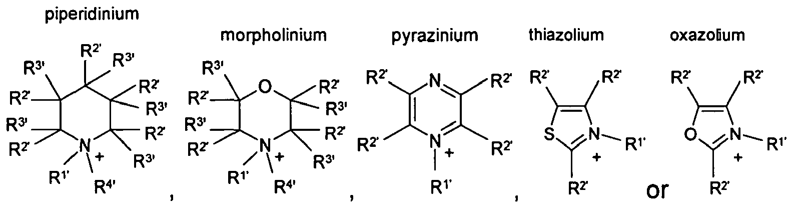 where the substituents
R^{1,} to R^{4,} each, independently of one another, denote hydrogen or CN,
straight-chain or branched alkyl having 1-20 C atoms,
straight-chain or branched alkenyl having 2-20 C atoms and one or more double bonds,
straight-chain or branched alkynyl having 2-20 C atoms and one or more triple bonds,
dialkylamino containing alkyl groups having 1-4 C atoms, which, however, is not bonded to the heteroatom of the heterocycle, saturated, partially or fully unsaturated cycloalkyl having 3-7 C atoms, which may be substituted by alkyl groups having 1-6 C atoms,
or aryl-C₁-C₆-alkyl,
where the substituents R^{1,} and R^{4,} may be partially or fully substituted by F, Cl and/or Br, in particular F and/or Cl, but where R^{1'} and R^{4'} are not simultaneously CN or cannot simultaneously be fully substituted by F or other halogens,
where the substituents R^{2'} and R^{3'} may be partially or fully substituted by F, Cl and/or Br, in particular F and/or Cl, or partially by NO₂ or CN and where, in the substituents R^{1,} to R^{4,}, one or two non-adjacent carbon atoms which are not bonded directly to the heteroatom and are not in the ω-position may be replaced by atoms and/or atom groups selected from the group -O-, -C(O)-, -S-, -S(O)- or -SO₂-.

10. Process according to one or more of Claims 1 to 9, **characterised in that** the reaction of the halide with the dialkyl sulfite is carried out without a solvent.

11. Use of onium alkylsulfites of the formula (9), obtainable by the process according to Claim 1
[Kt]⁺ [alkyl-O-SO₂]⁻ (9)
where Kt denotes asymmetrical tetraalkylammonium, symmetrical and asymmetrical phosphonium, guanidinium, thiouronium cations or heterocyclic cations, as starting material for the synthesis of onium salts with alkylsulfate anions by oxidation.

12. 1-Butyl-3-methylimidazolium ethylsulfite, 1-ethyl-3-methylimidazolium ethylsulfite.

## Revendications

1. Procédé de préparation d'alkylsulfites d'onium par la réaction d'un halogénure ou carboxylate d'onium avec un sulfite de dialkyle symétriquement substitué ou avec un sulfite de dialkyle asymétriquement substitué à des températures allant de 0 à 70°C.

2. Procédé selon la revendication 1, **caractérisé en ce que** la réaction est effectuée avec un sulfite de dialkyle symétriquement substitué, où le groupement alkyle peut avoir de 1 à 10 atomes de C.

3. Procédé selon la revendication 1, **caractérisé en ce que** la réaction est effectuée avec un sulfite de dialkyle asymétriquement substitué, où l'un des groupements alkyle peut avoir de 1 à 10 atomes de C et le deuxième groupement alkyle désigne méthyle ou éthyle.

4. Procédé selon l'une ou plusieurs parmi les revendications 1 à 3, **caractérisé en ce que** l'halogénure est un halogénure d'ammonium, un halogénure de phosphonium, un halogénure de thiouronium, un halogénure de guanidinium ou un halogénure ayant un cation hétérocyclique, où les halogénures peuvent être choisis dans le groupe constitué par les fluorures, les chlorures ou les bromures.

5. Procédé selon l'une ou plusieurs parmi les revendications 1 à 4, **caractérisé en ce que** le carboxylate est un acétate d'ammonium, un acétate de phosphonium, un acétate de guanidinium ou un acétate ayant un cation hétérocyclique.

6. Procédé selon l'une ou plusieurs parmi les revendications 1 à 4, **caractérisé en ce que** l'halogénure répond à la formule (1),
[XR₄]⁺ Hal⁻ (1),
dans laquelle
X désigne N, P
Hal désigne F, Cl ou Br et
R désigne dans chaque cas, indépendamment l'un de l'autre, H, où tous les substituants R ne peuvent être simultanément H,
alkyle à chaîne linéaire ou ramifiée ayant 1-20 atomes de C,
alcényle à chaîne linéaire ou ramifiée ayant 2-20 atomes de C et une ou plusieurs doubles liaisons,
alcynyle à chaîne linéaire ou ramifiée ayant 2-20 atomes de C et une ou plusieurs triples liaisons,
cycloalkyle saturé, partiellement ou totalement insaturé ayant 3-7 atomes de C, qui peut être substitué par des groupements alkyle ayant 1-6 atomes de C,
où un ou plusieurs R peuvent être partiellement substitués par Cl, Br et/ou CN ou partiellement ou totalement par F, ou F et Cl, ou F et Br, ou F, CI et Br, mais où tous les quatre ou trois R ne peuvent être totalement substitués par des halogènes,
et où un ou deux atomes de carbone non adjacents de R qui ne sont pas en position α ou ω peuvent être remplacés par des atomes et/ou des groupes d'atomes choisis dans le groupe constitué par -O-, -C(O)-, -S-, -S(O)- ou -SO₂-.

7. Procédé selon l'une ou plusieurs parmi les revendications 1 à 4, **caractérisé en ce que** l'halogénure répond à la formule (2),
[C(NR¹R²)(NR³R⁴)(NR⁵R⁶)]⁺ Hal⁻ (2),
dans laquelle
Hal désigne F, Cl ou Br et
R¹ à R⁶ désignent chacun, indépendamment l'un de l'autre,
hydrogène ou CN,
alkyle à chaîne linéaire ou ramifiée ayant 1 à 20 atomes de C,
alcényle à chaîne linéaire ou ramifiée ayant 2-20 atomes de C et une ou plusieurs doubles liaisons,
alcynyle à chaîne linéaire ou ramifiée ayant 2-20 atomes de C et une ou plusieurs triples liaisons,
cycloalkyle saturé, partiellement ou totalement insaturé ayant 3-7 atomes de C, qui peut être substitué par des groupements alkyle ayant 1-6 atomes de C,
où un ou plusieurs parmi les substituants R¹ à R⁶ peuvent être partiellement substitués par NO₂, CN, CI et/ou Br, partiellement ou totalement par F, ou F et Cl, ou F et Br, ou F, Cl et Br, mais où tous les substituants sur un atome de N ne peuvent être totalement substitués par des halogènes,
où les substituants R¹ à R⁶ peuvent être reliés l'un à l'autre en paires par une liaison simple ou double et
où, dans les substituants R¹ à R⁶, un ou deux atomes de carbone non adjacents qui ne sont pas liés directement à l'hétéroatome et ne sont pas en position ω peuvent être remplacés par des atomes et/ou des groupes d'atomes choisis dans le groupe constitué par -O-, -C(O)-, -S-, -S(O)- ou -SO₂-.

8. Procédé selon l'une ou plusieurs parmi les revendications 1 à 4, **caractérisé en ce que** l'halogénure répond à la formule (3),
[(R¹R²N)-C(=SR⁷)(NR³R⁴)]⁺ Hal⁻ (3),
dans laquelle
Hal désigne Cl ou Br et
R¹ à R⁴ et R⁷ désignent chacun, indépendamment l'un de l'autre, hydrogène ou CN, où hydrogène est exclu pour R⁷,
alkyle à chaîne linéaire ou ramifiée ayant 1 à 20 atomes de C,
alcényle à chaîne linéaire ou ramifiée ayant 2-20 atomes de C et une ou plusieurs doubles liaisons,
alcynyle à chaîne linéaire ou ramifiée ayant 2-20 atomes de C et une ou plusieurs triples liaisons,
cycloalkyle saturé, partiellement ou totalement insaturé ayant 3-7 atomes de C, qui peut être substitué par des groupements alkyle ayant 1-6 atomes de C,
où un ou plusieurs parmi les substituants R¹ à R⁴ et R⁷ peuvent être partiellement ou totalement substitués par F, CI et/ou Br, en particulier F et/ou Cl, ou partiellement par CN, mais où tous les substituants sur un atome de N ne peuvent être totalement substitués par des halogènes, où les substituants R¹ à R⁴ et R⁷ peuvent être reliés l'un à l'autre en paires par une liaison simple ou double et où, dans les substituants R¹ à R⁴ et R⁷, un ou deux atomes de carbone non adjacents qui ne sont pas liés directement à l'hétéroatome et ne sont pas en position ω peuvent être remplacés par des atomes et/ou des groupes d'atomes choisis dans le groupe constitué par -O-, -C(O)-, -S-, -S(O)- ou -SO₂-.

9. Procédé selon l'une ou plusieurs parmi les revendications 1 à 4, **caractérisé en ce que** l'halogénure répond à la formule (4),
[HétN]⁺ Hal⁻ (4),
dans laquelle
Hal désigne Cl ou Br et
HétN⁺ désigne un cation hétérocyclique choisi dans le groupe constitué par 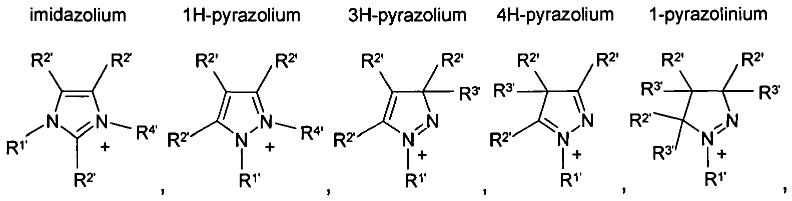 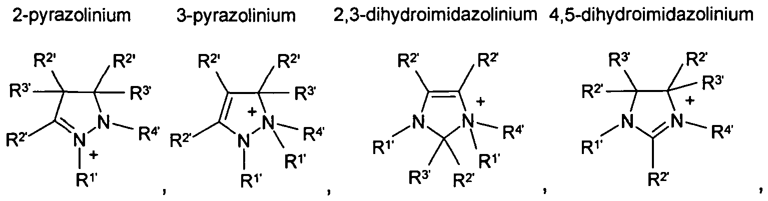 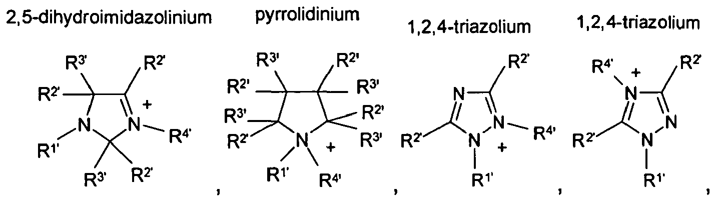 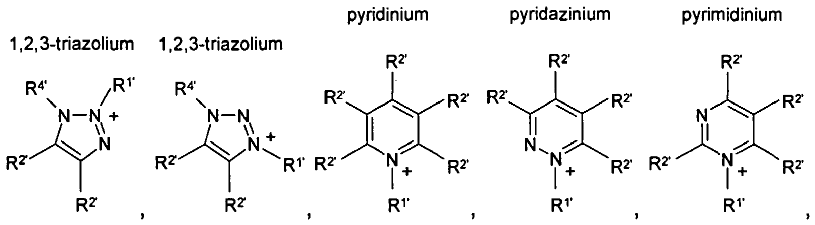 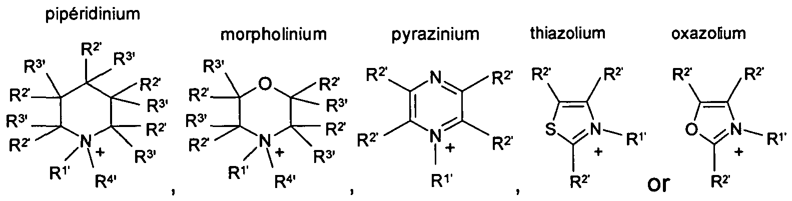 où les substituants
R^{1,} à R⁴' désignent chacun, indépendamment l'un de l'autre, hydrogène ou CN,
alkyle à chaîne linéaire ou ramifiée ayant 1-20 atomes de C,
alcényle à chaîne linéaire ou ramifiée ayant 2-20 atomes de C et une ou plusieurs doubles liaisons,
alcynyle à chaîne linéaire ou ramifiée ayant 2-20 atomes de C et une ou plusieurs triples liaisons,
dialkylamino contenant des groupements alkyle ayant 1-4 atomes de C, qui, cependant, n'est pas lié à l'hétéroatome de l'hétérocycle, cycloalkyle saturé, partiellement ou totalement insaturé ayant 3-7 atomes de C, qui peut être substitué par des groupements alkyle ayant 1-6 atomes de C,
ou aryl-C₁-C₆-alkyle,
où les substituants R^{1,} et R^{4,} peuvent être partiellement ou totalement substitués par F, CI et/ou Br, en particulier F et/ou Cl, mais où R^{1'} et R^{4'} ne sont pas simultanément CN ou ne peuvent être simultanément totalement substitués par F ou d'autres halogènes,
où les substituants R^{2'} et R^{3'} peuvent être partiellement ou totalement substitués par F, Cl et/ou Br, en particulier F et/ou CI, ou partiellement par NO₂ ou CN
et où, dans les substituants R^{1'} à R^{4'}, un ou deux atomes de carbone non adjacents qui ne sont pas liés directement à l'hétéroatome et ne sont pas en position ω peuvent être remplacés par des atomes et/ou des groupes d'atomes choisis dans le groupe constitué par -O-, -C(O)-, -S-, -S(O)- ou -SO₂-.

10. Procédé selon l'une ou plusieurs parmi les revendications 1 à 9, **caractérisé en ce que** la réaction de l'halogénure avec le sulfite de dialkyle est effectuée sans solvant.

11. Utilisation d'alkylsulfites d'onium de formule (9), pouvant être obtenus par le procédé selon la revendication 1
[Kt]⁺ [alkyl-O-S0₂]⁻ (9)
où Kt désigne des cations hétérocycliques ou des cations de phosphonium, guanidinium, thiouronium symétriques et asymétriques, de tétraalkylammonium asymétrique, comme produit de départ pour la synthèse de sels d'onium avec des anions d'alkylsulfate par oxydation.

12. Ethylsulfite de 1-butyl-3-méthylimidazolium, éthylsulfite de 1-éthyl-3-méthylimidazolium.
